(19) Europäisches Patentamt
European Patent Office
Office européen des brevets

(11) Publication number : 0 445 914 A1

# (12) EUROPEAN PATENT APPLICATION

(21) Application number : 91300777.9

(22) Date of filing : 31.01.91

(51) Int. Cl.$^5$ : A61K 7/46

(30) Priority : 02.02.90 US 474564
02.02.90 US 474565

(43) Date of publication of application :
11.09.91 Bulletin 91/37

(84) Designated Contracting States :
DE FR GB NL

(71) Applicant : HERCULES INCORPORATED
1313 N. Market Street, Hercules Plaza
Wilmington, Delaware 19894-0001 (US)

(72) Inventor : Hussain, Zahera Jabeen
337 Quail Ridge Road
Hyde Park, New York 12538 (US)
Inventor : Lawter, Louise Marie
35 Glen Drive
Goshen, New York 10924 (US)
Inventor : Sherman, Gregory Alan
R.D. No. 3, Box 155
Middletown, New York 10940 (US)

(74) Representative : De Minvielle-Devaux, Ian
Benedict Peter et al
CARPMAELS & RANSFORD 43, Bloomsbury
Square
London WC1A 2RA (GB)

## (54) Carrier composition and method.

(57) A composition and method of making it. The composition includes a carrier and particles adhered to the carrier. The particles include oil and water soluble matrix material. The encapsulation material is water soluble. The oil is encapsulated by the encapsulation material. The carrier is substantially insoluble in the water.



Jouve, 18, rue Saint-Denis, 75001 PARIS

# CARRIER COMPOSITION AND METHOD

The invention relates to a composition including a carrier and particles of oil encapsulated in encapsulation material. In particular, the invention relates to adhering the particles to the carrier.

Brenner et al., U.S. Patent 3,971,852, disclose a process of microencapsulating an oil in a water soluble matrix material. Zelter, in U.S. Patent 4,746,567, discloses a paper product for storing fragrances. The paper has embedded microcapsules which are crushed to release fragrance.

There is a need for a composition having a carrier and particles of encapsulated oil adhered thereto which is adapted to provide initial rapid release of the oil upon contact with water as is provided by the present invention. Accordingly the invention provides a composition including a carrier and particles adhered thereto of water soluble encapsulation material and oil. The release of the oil, such as fragrance, from the particles adhered to the carrier is initiated by contact with water (moisture). The fragrance then evaporates into the atmosphere surrounding the carrier.

The invention provides in a composition having a carrier substantially water-insoluble characterized in that the carrier is substantially adhered to particles of encapsulated oil and water soluble hydrophilic encapsulation matrix material. In a preferred embodiment of the invention, the carrier includes a sheet of cellulosic paper. Carriers may have any desired shape such as rectangular, disk, spherical, cylindrical or ornamental, for example, having the shape of a flower. Alternatively, the carrier may include non wovens or functional cloth items fabricated from nonwovens such as gloves, aprons, gowns. Water absorbent materials such as pads, cloth, tissues and towels may also be used as carriers. The carrier supports particles of encapsulated oil. The encapsulated oil particle preferably consists of an oil and a hydrophilic matrix material. The encapsulation matrix material is preferably water soluble.

Particles, dispersed in a dispersion medium, are preferably incorporated by forming a dispersion in a liquid, for example, a non-polar liquid, such as branched esters of palmitic, myristic, stearic and adipic acids; cyclic siloxanes such as cyclomethicone pentamer, tetramer and hexamer that are commonly known as volatile silicones or emollient oils, such as light mineral oil, jo jo ba oil, aloe vera.

Preferably, the oil encapsulated is a volatile oil, for example, natural and synthetic essential oils or compounded fragrance oils such as citrus (orange, lemon, lime and the like), spice oils (cascia, clove, wintergreen and the like), mint oils, (spearmint, peppermint, and the like), woody oils (vetiver, patchouli, and the like); perfume oils and individual components thereof, such as linalool, methyl salicylate, limonene, methol, decanol, diethyl phthalate, carvone, citral, and the like). Other oils such as insectrepellents, antimicrobials, bactericides and flavor or fragrance oils, may also be encapsulated and supported by a carrier in accordance with the invention. The liquid containing the particles of encapsulated oil is absorbed by the carrier through wetting and capillarity. This enables the dispersed particles to be entrapped by the interstices of the carrier thereby favoring adhesion.

Particles of encapsulated oil are preferably blended into an absorbent or emollient powder such as talc, calcium carbonate, sodium bicarbonate. The blend is then sprayed onto the carrier while the carrier is partially moist for uniform adhesion of the powder.

A preferred method of supporting the particles of encapsulated oil by the carrier is to heat treat the carrier to the softening point of the encapsulating material so that adhesion occurs via melting. The particles are then applied to the carrier for example, alone or as part of surfactant and powder blend, utilizing a pressurized spraying system. Upon cooling, the particles are affixed to the carrier.

To affix particles to the carrier, a tackifier is used. Preferred tackifiers include waxes either alone or dissolved in a solvent such as acetone. Tackifiers are applied, for example, by spraying onto encapsulation particles to provide surface modified particles having improved adhesion to carriers. An example of surface modified particles is a wax coated particle of encapsulated oil.

The encapsulation (matrix) materials include those in U.S. Patent 3,971,852 and lipophilic starch derivatives in combination with polyhydroxyalcohols that have moisture release mechanism. Variation of encapsulation materials is used to vary the rate of release of fragrance. Differences in the melting point film forming ability and or hydrophobicity of the encapsulation (matrix) material are selected to control the rate of release of fragrance. By combining particles having different matrix materials onto a carrier a continuous release of fragrance is provided. The particle may be a microencapsulated or macroencapsulated fragrance.

Preferred encapsulation materials are characterized by their ability to for a continuous phase of an emulsion in which a high proportion of oil can be held as the dispersed phase. These preferred encapsulation materials are also characterized by plasticity or flowability in the drying temperature range during which a solid is derived from the emulsion by removal of moisture, e.g., when the emulsion is converted into particles by a spray drying or other drying procedure. Encapsulation (matrix) materials include mixtures of polysaccharides and polyhydroxy compounds which form emulsions with the oil. The matrix forming materials in these emulsions have a plastic or flowable state over a substantial portion of the critical temperature range over which water is readily removed between the fully liquid and fully solid states. They also form a surface that selectively permits removal of water and they become, on removal of moisture, a cellular matrix of the polysaccharide and polyhydroxy materials in solid state with oil fixed in the cells thereof. Materials other than polysaccharides and polyhydroxy compounds that, alone or in combination, satisfy these criteria may be substituted for part or all of one or both of these materials.

The polysaccharides employed in encapsulation materials in admixture with polyhydroxy compounds to encapsulate oil such as fragrance oil are solids characterized by solubility in water and by at least partial solubility in, or capability of at least partially dissolving, the polyhydroxy compound within the ranges of proportions used. They are primarily not the sweet, readily soluble saccharides like sugar but higher polysaccharides that may be natural, such as gum arabic and similar vegetable gums, or synthetic, such as degradation and modified products of starch, which usually form colloidal solutions. Certain starch degradation products such as dextrinized starch which are suitable polysaccharides for use in system and process of the invention contain a wide spectrum of saccharides of different molecular weights including a sufficient proportion in thepolysaccharide molecular weight range to be good encapsulants and varying proportions of lower saccharides such as mono-, di- and trisaccharides which are polyhydroxy compounds, as later defined. When such polysaccharides are used it may be necessary to make adjustments in the proportion of added polyhydroxy compound in order to obtain the proper balance of polysaccharide and polyhydroxy compounds to assure proper melt characteristics as later described since the proportion of lower saccharides in the starch degradation products, while usually too low to satisfy the requirements of the invention for polyhydroxy compound, may be large enough to affect significantly the amount of added polyhydroxy compound required to obtain the said proper proportion of polyhydroxy compound to polysaccharide in the product.

Among the polysaccharides employed in encapsulation material are dextrins derived from ungelatinized starch acid esters of substituted dicarboxylic acids represented diagrammatically by the formula:

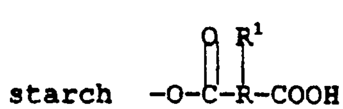

in which R is a radical selected from the class consisting of dimethylene and trimethylene and $R^1$ is a hydrocarbon substituent of R selected from the class consisting of alkyl, alkenyl, aralkyl and aralkenyl groups. These ungelatinized starch-acid esters are prepared by reacting an ungelatined starch, in an alkaline medium, with a substituted cyclic dicarboxylic acid anhydride having the following formula:

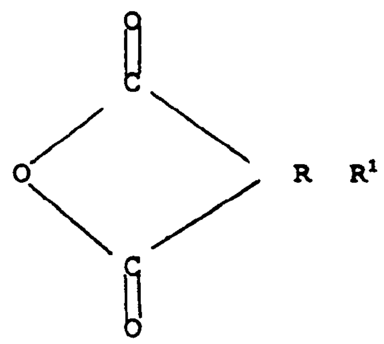

in which R and $R^1$ represent the so designated substituent groups just defined. Examples of such anhydrides are the substituted succinic and glutaric acid anhydrides. Other useful polysaccharides include products derived from dextrinized starch, and hydrolyzed starch. In general, these products contain minor proportions of lower saccharides such as dextrose. The polysaccharide content may comprise a single polysaccharide or mixture of two or more polysaccharides.

The polysaccharide for use in encapsulation material preferably possesses emulsifying properties either inherently or by reason of the presence of a minor proportion of a suitable emulsifying agent. Further definition of emulsifying agents is unnecessary because they are well known to those skilled in the art. Examples of satisfactory emulsifying agents are sodium diisooctyl sulfosuccinate and sodium caseinate. If emulsifying agents are added, proportions in the range of 0.1 to 10% based on the weight of polysaccharide in the mixture are satisfactory.

Preferably, the polyhydroxy compounds are employed in encapsulation material in admixture with polysaccharide material as the encapsulation material for encapsulated fragrance. Such compounds are characterized by solubility in water and at least partial solubility in the polysaccharide material or capability of at least partially dissolving such material. Polyhydroxy compounds form with the polysaccharide material into a liquid melt which softens within the range of from 30 to 100°F for the ranges of proportions used. With the polysaccharide material the polyhydroxy compounds form a continuous aqueous phase in which oil is dispersible as a discontinuous phase to form a stable emulsion. The plasticity of the surface of the particles formed from the emulsion diminishes as water is removed through a drying operation, and the particles formed are in a solid state at the temperature of use. Preferred polyhydroxy compounds for use in encapsulation material can be classified in these groups: alcohols, sugars from plant sources and those containing other functional groups. Polyhydroxy alcohols include glycerine, sorbitol, mannitol, erythritol and ribitol. The sugars from plant sources, include monosaccharides such as glucose, disaccharides such as maltose and sucrose, trisaccharides such as raffinose, and ketosaccharides such as fructose. These will be referred to as plant-type sugars whether actually derived from plants or produced synthetically. Polyhydroxy compounds containing other functional groups include glucuronolactone (lactone), sorbitan and mannitan (monoethers) and methyl-glucopyranoside (acetal).

The fragrance oils that can be encapsulated in accordance with the present invention include nonvolatile as well as volatile oils. The oils are characterized by being insoluble but dispersible (emulsifiable) in water and they may be volatile or nonvolatile under drying conditions which include elevated temperature and low relative humidity in the air stream. They are usually liquid at the temperature of the emulsion but petroleum jelly can be successfully encapsulated for use in the process of the invention since it is readily broken up into tiny particles in an emulsifying machine producing high shear.

The proportions of oil to encapsulate in the encapsulated fragrance matrix may vary widely from small but effective amounts. Typically, the fragrance amounts to from about 5 to about 80 percent by volume of the encapsulated fragrance matrix. High yield and low extractable oil are realized in greatest measure where the oil amounts to at least 30% by volume of the encapsulated fragrance matrix.

Wax as used throughout this disclosure includes natural waxes such as beeswax, synthetic waxes such as polyethylene waxes, esters of fatty acids, vegetable waxes such as candelilla wax, carnauba wax, ozokerite wax and paraffin waxes.

Non-polar liquids, as used throughout this disclosure, preferably have a low viscosity of from one to ten centipoise (cps Brookfield at 20°C), a low vapor pressure and a low boiling point from 60-300°C. Most preferably the non-polar liquids used in accordance with the invention have a boiling point of from 95-110°C.

In a preferred embodiment, encapsulation particles dispersed in a non-polar liquid are applied to a carrier, which is hydrophilic and absorbent. Hydrophobic carriers are preferably treated with surfactant to improve the wetting thereof.

In the Examples below and throughout this disclosure, percentages are by weight unless otherwise specified.

Example 1

A solution of an encapsulant comprising 32 parts glucoronolactone and 48 parts polysaccharide X is prepared by dissolving them in 250 parts of water with agitation at high speed in a household type Waring blender. Single fold orange oil containing 1% butylated hydroxy anisole as antioxidant is slowly added to the resulting solution until 120 parts are incorporated while continuing high speed agitation for 3 minutes, at which time an oil/water emulsion had formed with an average droplet diameter of 0.5 microns. The viscosity as determined with a Brookfield Model LVT Viscometer is 57.5 centipoise at 30°C. The proportions are chosen to give an oil loading of 60% (120 parts oil and 80 parts encapsulant). The mixture is spray dried in a standard Anhydro laboratory drier, size No. 1, maintained at an air inlet temperature of 180°C and an air outlet temperature of 90°C at a feed rate of 3 lbs per hour of emulsion. There is collected 170 parts of powdered product readily passing through a 140 mesh screen which upon analysis by standard steam distillation technique is shown to contain 66% by volume (V/W) or 56% by weight (W/W) of volatile oil based on the weight of the product. This represents an 85% weight recovery of product containing 93% of the theoretical load of orange oil initially employed to make the emulsion. This represents a total recovery of 79% of the original oil. The extractable oil of the product is 0.2% as determined by extraction as described above. The moisture content is 2.1% as determined by Karl Fisher procedure. In general the volatile oil content is determined by the standard steam distillation technique on product as produced. The volatile oil content as so determined includes the extractable oil. The dry powder is then added at 1.0% by weight with 1.26% fatty acid esters of sorbitol anhydrides (Span 85) to aerosol propellent and sprayed onto a sheet of paper towel to provide 0.03 gram encapsulation per 5 gram sheet. Release is activated by wetting with water. The original odor profile and strength is preserved.

Example 2

An emulsion is prepared from 32 parts of sorbitol, 48 parts of gum arabic, 120 parts of an orange oil, 2 parts of sodium di isooctyl sulfosuccinate and 300 parts of water. The resulting emulsion has an average oil particle size of 1.4 microns and a viscosity of 40 centipoise at 30°C. The spray dried powder obtained in a weight yield of 80.3% has 67.4% volatile oil (57.2% by weight, oil factor 0.95) and 0.9% moisture. The product dissolves readily in cold water. Comparable results are obtained using mannitol and sucrose instead of sorbitol. This product powder is then sprayed using hot air as a vehicle onto paper tissue to provide 0.03 grams of particles per sheet of paper tissue. Each sheet of paper tissue having a mass of 2 grams. Release is activated by wetting the paper tissue with water. The original odor profile and strength is preserved.

Example 3

Product particles formed in accordance with example 2 are sprayed onto a paper sheet after forming during the drying step of a paper making process as follows. After the water is removed during the drying of the paper sheet, the particles are sprayed onto the hot sheet. The heat in the sheet melts the encapsulating material. The melted encapsulating material adheres to the sheet. Upon cooling, the encapsulated particles are affixed to the sheet. Fragrance odor on the dry sheet is negligible. Fragrance is released upon subsequent contact with moisture.

Example 4

Particles formed according with Example 2 are affixed to absorbent pads in order to achieve "on demand" fragrance release as follows. The absorbent carrier pads are dried at to 70°C. The powder is sprayed onto dry absorbent carrier of cellulosic fibers of the pad. The pad is cooled. As the encapsulating material cools, the encapsulated fragrance particles become affixed to the carrier. Thus, the powder is prevented from being shaken out of the pad during packaging and storage.

Example 5

Particles are affixed to absorbent carrier pads as in Example 4 except that the particles are sprayed onto the carrier before the carrier is heated.

Example 6

The powder made in accordance with Example 1 is then fluidized in a fluidized bed. Melted wax at 80°C is sprayed onto the fluidized powder. The wax forms a monomolecular wax coating on the particles as the wax cools upon contacting the particles. The wax coated particles are then sprayed onto warm (60°C) tissue paper. The paper is cooled to 20°C and the coated particles adhere thereto.

Other features, advantages and specific embodiments of this invention will become readily apparent to those exercising ordinary skill in the art after reading the foregoing disclosures. In this regard, while specific embodiments of this invention have been described in considerable detail, variations and modifications of these embodiments can be effected without departing from the spirit and scope of the invention as disclosed and claimed.

**Claims**

1. A composition comprising a substantially water-insoluble carrier and encapsulated particles applied thereto, characterized in that the particles comprise oil and hydrophilic water soluble encapsulation matrix material, said oil being encapsulated in said material, and said particles being adhered to said carrier.

2. The composition of claim 1 wherein said particles are coated with tackifier.

3. The composition of claim 1 or 2 wherein said carrier comprises paper, nonwoven, fiber or absorbent pad.

4. The composition of any of claims 1-3 wherein said oil comprises 5 to 98% by volume of said particles.

5. The composition of any of claims 1-4 wherein said oil comprises fragrance oil, flavor oil, a natural essential oil, a synthetic essential oil, compounded fragrance oil, perfume oil, insect repellent or individual components thereof.

6. The composition of claim 5 wherein said encapsulation matrix material is a solid matrix comprising a mixture of a modified starch derived from ungelatinized starch acid esters of substituted dicarboxylic acids represented by the formula:

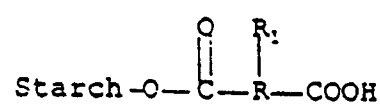

in which R is dimethylene or trimethylene and $R_1$ is alkyl, alkenyl, aralkyl or aralkenyl groups and a polyhydroxy compound present in an amount at least 10% by weight of the mixture and selected from plant-type sugars, lactones, monoethers and acetals.

7. The composition of claim 5 wherein said encapsulation matrix material comprises a mixture of a hydrolyzed starch and a polyhydroxy compound present in an amount at least 10% by weight of the mixture, said polyhydroxy compound being selected from the group consisting of alcohols, plant-type sugars, lactones, monoethers and acetals.

8. A process for producing the composition of any of claims 1-7 wherein the carrier is substantially adhered to the particles by dispersing the particles in a dispersion medium to form a dispersion, and applying the dispersion to the carrier.

9. A process for producing the composition of any of claims 1-7 wherein the carrier is substantially

adhered to the particles by physically connecting the particles to the carrier.

10. A process for producing the composition of claim 6 or 7 wherein the encapsulated particles are formed by emulsifying the oil in a solution of the modified or hydrolyzed starch and polyhydroxy compound in water, and spray drying the emulsion to remove water therefrom.

11. A process for producing the composition of any of claims 1-7 wherein the particles are adhered to said carrier by heating the carrier to a temperature at which the encapsulating material softens and contacting the particles with the heated carrier whereby adherence is effected.

12. The process of claim 11 wherein the carrier is cooled while particles are in contact therewith whereby the particles are affixed to the carrier.

13. The process of claim 11 or 12 wherein the said particles are coated with tackifier by spraying tackifier into a fluidized bed of the particles.

14. A process for producing the composition of any of claims 1-7 wherein the particles are substantially adhered to the carrier by dispersing the particles in a dispersion medium to form a dispersion, and applying the dispersion to the carrier.

European Patent Office

# EUROPEAN SEARCH REPORT

Application Number

EP 91 30 0777

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DATABASE WPIL, accession no. 78-59536A [33], Derwent Publications Ltd, London, GB; & JP-A-53 079 611 (MITSUBISHI PAPER MILLS)<br>* Abstract * | 1 | A 61 K 7/46 |
| A | WPIL / DERWENT, accession no. 82-23121E [12], Derwent Publications Ltd, London, GB; & JP-A-57 027 981 (MATSUSHITA ELEC. WORKS)<br>* Abstract * | 1,8,9 | |
| A,D | US-A-3 971 852 (J.J. BRENNER et al.)<br>* Column 4, lines 40-68; column 5, lines 1-16; column 6, lines 2-40; column 10, lines 26-47 * | 1,5,6,7,10 | |
| A | DATABASE WPIL, accession no. 80-360970, Derwent Publications Ltd, London, GB; & JP-A-1 270 937 (KAO CORP.)<br>* Abstract * | 1 | TECHNICAL FIELDS SEARCHED (Int. Cl.5) |
| A | GB-A-1 273 322 (THE NATIONAL CASH REGISTER CO.)<br>* Page 3, lines 95-115 * | 8,9,14 | B 01 J |

The present search report has been drawn up for all claims

| Place of search | Date of completion of the search | Examiner |
|---|---|---|
| THE HAGUE | 22-04-1991 | KERRES P.M.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)